# EUROPEAN PATENT SPECIFICATION

(11) **EP 4 226 873 B1**
(45) Date of publication and mention of the grant of the patent: **04.06.2025**
(21) Application number: 22155802.6
(22) Date of filing: 09.02.2022
(51) Int. Cl.: A61B 17/225, A61B 17/00

(54) **SHOCK WAVE DEVICE WITH MOTION COMPENSATION**
STOSSWELLENVORRICHTUNG MIT BEWEGUNGSKOMPENSATION
DISPOSITIF À ONDES DE CHOC AVEC COMPENSATION DE MOUVEMENT

(43) Date of publication of application: 16.08.2023
(73) Proprietor: Storz Medical AG, 8274 Tägerwilen (CH)
(72) Inventor: STORZ, Rafael, 8280 Kreuzlingen (CH); MARLINGHAUS, Ernst, 8598 Bottighofen (CH)
(74) Representative: Lohr, Jöstingmeier & Partner

(56) References cited:
- EP-A1- 2 628 456
- DE-A1- 10 260 594
- DE-C2- 3 943 644
- US-A- 4 763 652
- US-A- 4 984 575
- US-A1- 2009 275 866
- US-A1- 2016 114 194

## Description

### Field of the invention

The invention relates to an extracorporeal shock wave therapy system, e.g. a lithotripsy system or Lithotripter for non-invasive treatment of stones, like e.g. kidney stones, urinary stones, gallstones or other calculi within a mammal's body using acoustic pulses, or other applications of an extracorporeal shock wave therapy system where the treatment area is in motion during treatment.

### Description of the related art

A device and a method for localizing stones and for targeting a shock wave source of a lithotripter is disclosed in EP 2 340 781 A1. Here a shockwave generator, a patient table and an ultrasound targeting device are provided with optical markers for tracking with a pair of infrared cameras. The body or parts of the body of a patient move slightly during breathing. This causes kidneys and other organs to move slightly such that they may move out of the focal volume of a shock wave source.

DE 36 21 935 discloses a lithotripter which is synchronized to the signal of a respiration sensor. This allows only for short treatment periods with a comparatively low repetition frequency corresponding to the respiration frequency.

EP 2 628 456 A1 discloses a shock wave therapy device with dynamic target tracking. US 2016/0114194 A1 discloses an optimized therapeutic energy delivery.

### Summary of the invention

The problem to be solved by the invention is to provide a shock wave device, which allows treatment of organs or areas within organs independent of respiration. The shock wave device should be comparatively simple and avoid complex and expensive mechanics.

Solutions of the problem are described in the independent claim.

The dependent claims relate to further improvements of the invention.

### Embodiments of the disclosure

In an embodiment, a shock wave device, which may be a lithotripter includes a shock wave source and may further include a patient table. The shock wave source may be of any type suitable for generating shock waves. It may include a shock wave generator and/or transducer, which may include at least one of a coil, a spark gap or a Piezo transducer. The shock wave generator/transducer may be partially enclosed by a reflector. Depending on the type of transducer, the reflector may have a parabolic or half-elliptic shape. The shock wave source may have a focal volume which is distant from the shock wave source and normally around a center axis of the shock wave source. The focal volume may be defined as a volume, where the maximum shock wave intensity is maintained with a deviation of maximal -3 dB or -6 dB. If the focal volume is defined with a 6 dB deviation, the pressure at the limit of the zone is half of the maximum pressure inside the zone. The focal volume may have an elliptical shape with a length in an axial direction (defined by the center axis) of the shock wave source axis of 10 to 15 cm and a diameter between 5 and 15 mm. The focal volume normally is spaced from the shock wave generator and/or transducer.

The patient table may have a basically planar surface defining a longitudinal axis. It is configured for accommodating a patient. The shock wave source may be mounted below the patient table. In general, a shockwave source may be mounted in alternative ways, e.g. on a stand or support.

Due to movements of a patient's body, e.g. by respiration or heartbeat, an object to be treated may move out of the focal volume. For example, a movement of the chest causes a movement of the kidneys in a cranio-caudal direction, which is approximately parallel to the surface of the patient table accommodating the patient. The amplitude of displacement of the kidneys normally is in the range of 20 to 40 mm. As the center axis of a normal shock wave generator is approximately orthogonal to the surface of the patient table, a kidney stone may easily move out of the focal volume. The respiration frequency of an anesthetized person may be in the range of 0.3 to 0.5 Hz.

In an embodiment, a shock wave source is arranged tiltable around at least one tilt axis which may be approximately parallel to a plane defined by a surface of a patient table, where a deviation in parallelism may be of +/- 30°. Further, the tilt axis may be orthogonal to a longitudinal axis of the patient table, where deviation in orthogonality of +/- 30° may be possible. The maximum angle of tilt may be in a range between 1° and 20°. It may be in a range between 1° and 10° or between 2° and 5°. A number of tests have shown that a typical angle of 2° is sufficient to track typical stone movements, whereas for larger movements an angle of 4° may be sufficient. Tilting of the shock wave source may be achieved by tilting the whole shock wave source including the shock wave generator/transducer and the reflector or only by tilting the shock wave generator/transducer. Tilting may be done by a tilting motor or a tilting drive, which may be a linear drive or any other suitable means. Tilting is synchronized to the motion of the patient body, which may be sensed by a motion sensor or by a respiration sensor. A respiration sensor may be a sensor configured for at least one of a chest movement of the patient due to respiration, a change of chest volume and/or size of the patient due to respiration, a change of position of an internal organ like kidney or heart by using ultrasound-imaging, a change of center of gravity of the patient and/or organ of interest which may be a kidney to be treated. Herein, for simplicity, such a sensor may be called a respiration sensor. The signal of the movement sensor/respiration sensor may be amplified with a variable gain. Further, an offset may be added to generate a tilt control signal defining the tilt angle. By this, a coupling of the tilt angle to the body movement and therefore to the movement of the kidney stone may be achieved.

As the position of the focal volume moves in synchronicity with the kidney stone, generating of shock wave pulses at any time will result in a high energy coupling into the stone and a high treatment efficiency.

For heart treatment, respiration and heartbeat may be considered. This may require a tilting movement about two different axes with different frequencies. Basically all parts of a body may be treated by compensating any movement by a tilting movement.

A fine adjustment of the amplitude of the tilting movement may either be made manually by a user who may watch the imaging system and an indication of the focal volume. Further, adjustment of the amplitude may be made automatically, for example by a computer system, analyzing the images of the imaging system.

A display may be provided indicating an image from an imaging system, like an x-ray image or and ultrasound image, which may further indicate the focal volume. Based on this, a user may estimate the quality of adjustment and treatment.

Due to the body movement or respiration tracking of the shock wave source, any shock wave repetition frequency, may be selected independent of the respiration frequency.

Due to the simple tilting movement of the shock wave source, only one movable axis is required, keeping the systems comparatively simple and efficient.

In an embodiment, the shock wave source may be rotated around the center axis about an angle in a range between +/- 2° to +/- 20°. The range may be between +/- 5° and +/- 15°. In a typical application, a range of +/-10° would meet most requirements. Usually, kidneys and kidney stones have a slightly outward movement in addition to the movement parallel to the patient table, which may be compensated by such a rotation around the center axis. In a very simple embodiment, there may be only two discrete angle settings, for example at + 10° and at - 10°, one for the left kidney and the other for the right kidney, which would suit most patients. In another embodiment, a larger number of positions may be provided. Alternately, a drive like a motor may be provided to adjust the angle.

The basic concept explains herein at the example of a kidney stone may also be applied to a urethra stone or other concrements in a body.

The shock wave device or lithotripter may also include a linear drive for the shock wave source, which may be operating in 1, 2 or 3 axes. Such a drive may be used for general adjustment of the relative position of the shock wave source relative to the patient.

The methods of operation disclosed are not explicitly recited by the wording of the claims but are considered useful for understanding the invention.

A method of focusing a shock wave source to a kidney stone comprises the steps of:
a) receiving a respiration signal indicative of a respiration,
b) amplifying the signal and optionally adding an offset,
c) tilting a shock wave source about an angle proportional to the signal.

The tilt axis may be an axis in a plane essentially parallel to a plane of a patient table and essentially orthogonal to a longitudinal axis of the patient table, including deviations in parallelism and/or orthogonality of +/- 30°.

The method may further include the steps of rotating the shock wave source around its center axis or a fixed angle before performing the steps a to c.

### Description of Drawings

In the following the invention will be described by way of example, without limitation of the general inventive concept, on examples of embodiment with reference to the drawings.
Figure 1 shows an embodiment.
Figure 2 shows a side view.
Figure 3 shows focal volume positions with tilting.
Figure 4 shows a top view.
Figure 5 shows a block diagram.

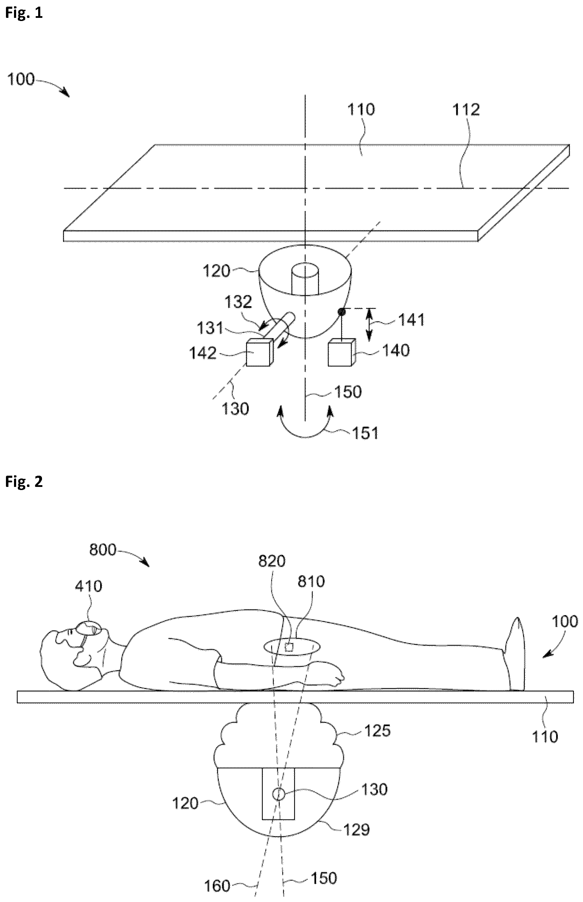

In Figure 1, a first embodiment is shown. A shock wave device, which may be a lithotripter 100 may include a patient table 110 and a shock wave source 120. The shock wave source 120 may be arranged below the patient table 110, such that a patient (not shown here) may be accommodated on top of the patient table. The patient table may have a longitudinal axis 112. There may be a hole or cutout in the patient table at the position of the shock wave source. The shock wave source 120 may be tiltable in a direction of tilt 132 around a tilt axis 130. The shock wave source may be held by a tilt shaft 131 and operated by a tilt drive motor 142. In another embodiment, a linear tilt drive 140 may be provided and configured for a linear movement in a linear tilt drive direction 141 to perform a tilt of the shock wave source 120. Basically, any kind of suspension and drive may be used, as long, as a tilt may be performed. This may also be a tripod drive. Further, the shock wave source 120 may have a center axis 150, and it may be rotatable in a rotation direction 151 around the center axis 150.

Figure 2 shows a side view. In this figure, a patient 800 is positioned on top of the patient table 110. The patient may have a kidney 810 with a kidney stone 820. Below the table 110, the shock wave source 120 is shown in a sectional view. It may have a shock wave generator 128 which may be a coil as shown herein and which is at least partially enclosed by a reflector 129. Further, the center axis 150 is marked as a dashed line and a tilted center axis 160 of a tilted state of the shock wave source is marked as a second dashed line. Normally, the interior of the reflector and the space between the source and the patient is filled with a liquid like water or another shockwave conducting medium. To contain the water within the volume, a coupling bellow 125 may be provided.

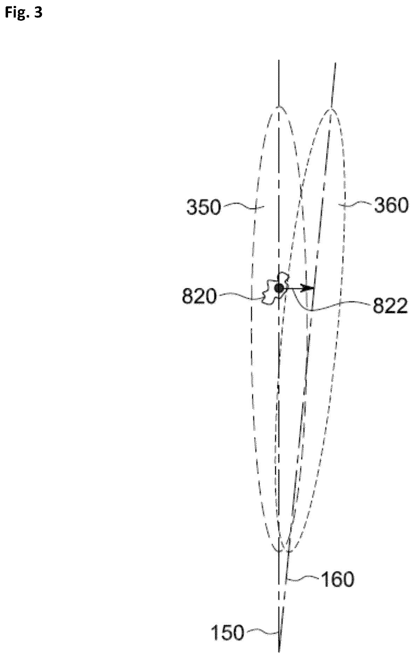

Figure 3 shows focal volume positions with tilting in a side view. On the left side, a first focal volume 350 is shown around the center axis 150 in a first state. When tilting the shock wave source about an angle, this focal volume is displaced to second focal volume 360 around a tilted center axis 160. In this figure, a tilt angle of approximately 4° is shown. It can be seen that the first focal volume from a normal state and the second focal volume from a tilted state together cover roughly twice the volume at the center of the focal volumes. A kidney stone 820, which may be centered to the center axis 150 in a first state, may move by respiration movement in a direction 822 to a second position, which then may be close to the tilted center axis 160. As the tilt of the shock wave source is synchronized with the respiration movement and therefore with the movement of the kidney 810 and with the movement of the kidney stone 820, the kidney stone is always approximately at the center axis of the shock wave source and therefore within the focal volume. This ensures a high energy coupling into the stone at any time.

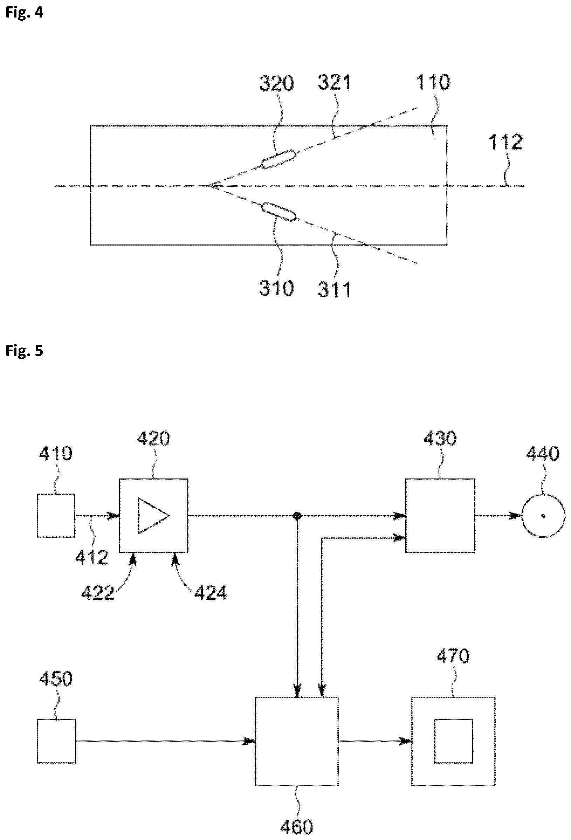

Figure 4 shows a top view. As the kidneys not only move roughly parallel to the longitudinal axis 112 of the body and therefore of the patient table, but also move slightly sidewards, the shock wave source may be rotated around the center axis 150 in a first direction, such that the tilt results in movement in a first tilt direction 311 under an angle to the longitudinal axis 112. Alternatively, the shock wave source may be rotated into an opposing second position, resulting in a tilting movement in a second tilt direction 321. The focal volume covered during the tilt movement is indicated with reference number 310 for the first tilt direction 311 and with reference number 320 for the second tilt direction 321.

Figure 5 shows a block diagram. A respiration sensor 410 produces a respiration signal or a signal related to respiration. Such a respiration signal may be a signal indicating the air flow into the body and/or out of the body, and/or a signal related to a movement of the body. For example, a sensor measuring the circumference of the chest may be used to generate a signal for such an indication. The signal is coupled into a signal amplifier 420 which may further receive a gain signal 422 and an offset signal 424. The gain signal and the offset signal may either be manually set by an operator and/or may be set by a controller/computer 460. This signal allows to modify the amplitude of the signal received by the respiration sensor and to add an offset if desired. The output signal of the signal amplifier 420 may be fed to a computer/controller 460. It may also be fed to a motor controller 430 which generates a control signal for a tilt motor 440. The tilt motor 440 may tilt the shock wave source around the tilt axis 130. As mentioned above, it may be a rotating motor or a linear drive, depending on the mechanical design. In an embodiment, there may be a linear relationship between the input signal into the motor controller 430 and the tilt angle, further resulting in a linear relationship between the respiration sensor signal and the tilt angle. To prevent excessive tilt angles, the motor controller 430 and/or the signal amplifier 420 may have a limiter to limit the output signal.

The controller/computer 460 may also receive a signal from an imaging system 450 which may be used to evaluate the position of the kidney stone. The controller/computer 460 may further be connected to a display 470 at which it may display an image from the imaging system 450 and/or an indication of the focal volume. The focal volume may be displayed in the same image, thus giving an indication of the overlapping of the focal volume with the kidney stone. This would allow to manually adjust the gain signal 422 and the offset signal 424 for best matching during respiration of the patient. The controller/computer 460 may also be connected to the motor controller 430 to control the motor e.g. based on the respiration sensor signal and/or a stone position derived from an image.

### List of reference numerals

- 100: lithotripter
- 110: patient table
- 112: longitudinal axis
- 114: table plane
- 116: axis orthogonal to table plane
- 120: shock wave source
- 125: coupling bellow
- 128: shock wave generator
- 129: reflector
- 130: tilt axis
- 131: tilt shaft
- 132: direction of tilt
- 140: linear tilt drive
- 141: linear tilt drive direction
- 142: tilt drive motor
- 150: center axis
- 151: rotation direction
- 160: tilted center axis
- 310: first focal volume coverage
- 311: first tilt direction
- 320: second focal volume coverage
- 321: second tilt direction
- 350: first focal volume
- 360: second focal volume
- 410: respiration sensor
- 412: respiration sensor signal
- 420: signal amplifier
- 422: gain signal
- 424: offset signal
- 430: motor controller
- 440: tilt motor
- 450: imaging system
- 460: controller/computer
- 470: display
- 800: patient
- 810: kidney
- 820: kidney stone
- 822: movement of kidney stone

## Claims

1. A shock wave device (100) comprising a shock wave source (120),
**characterized in, that**
the shock wave source (120) being coupled to a tilt drive (140, 142) and configured for a tilting movement (132) around at least one tilt axis (130), the tilt drive (140, 142) being coupled to a movement sensor (410) and configured for a tilting movement (132) as a function of a movement sensor signal (412) from the movement sensor (410), and the movement sensor (410) is at least one of a respiration sensor or a heart pulse sensor.

2. The shock wave device (100) according to claim 1,
**characterized in, that**
the shock wave device (100) is a lithotripter comprising a patient table (110), wherein the shock wave source (120) is arranged below the patient table (110),
the patient table (110) defining a table plane (114) and a longitudinal axis (112),
the shock wave source (120) is configured for a tilting movement (132) around a tilt axis (130) parallel to the table plane (114), and
the movement sensor (410) is a respiration sensor.

3. The shock wave device (100) according to any of the preceding claims,
**characterized in, that**
the tilting movement (132) is limited to an angle less than one of 20°, 10°, 5°, 4°, 2°.

4. The shock wave device (100) according to claim 2 or according to claim 3 in combination with claim 2,
**characterized in, that**
the shock wave source (120) is held by a tilt mount (131) which is further configured to rotate around an axis (116) orthogonal to the table plane (114) and/or
the tilt mount (131) is configured to be locked for a fixed rotation position when the tilt drive is operative.

5. The shock wave device (100) according to claim 2 or according to any of the claims 3-4 in combination with claim 2 relating to the respiration sensor (410),
**characterized in, that** the respiration sensor (410) is configured to be coupled to a patient (800), the patient being positioned on the patient table.

6. The shock wave device (100) according to claim 2 or according to any of the claims 3-5 in combination with claim 2,
**characterized in, that**
the shock wave source (120) is configured to generate shock waves propagating in a direction to the patient table (110) and having a focal volume (350) above the patient table (110).

7. The shock wave device (100) according to claim 2 or according to any of the claims 3-6 in combination with claim 2,
**characterized in, that**
the shock wave source (120) is configured to move the focal volume (350) by tilting parallel to the longitudinal axis (112) of the patient table (110) with a deviation caused by rotation.

8. The shock wave device (100) according to any of the preceding claims,
**characterized in, that**
the respiration sensor (410) is configured for sensing at least one of
- a respiration air flow in and/or out of the patient (800) coupled to a patient (800),
- a chest movement of the patient (800) due to respiration, and
- a change of chest volume and/or size of the patient (800) due to respiration
- a change of position of an internal organ like kidney or heart by using ultrasound-imaging,
- a change of center of gravity of an organ of interest.

9. The shock wave device (100) according to any of the preceding claims,
**characterized in, that** at least one imaging system (450) is provided and configured for generating images of a region of a patient.

10. The shock wave device (100) according to any of the preceding claims,
**characterized in, that** at least one controller/computer (460) is provided and configured to at least one of:
- calculating an optimal tilt angle and/or rotation angle,
- visualizing on a screen the position of an organ (810) and/or a stone (820) together with a focal volume (350),
- visualizing the movement of a focal volume (350) due to tilt,
- visualizing the total focal volume (310) covered by tilt.

## Patentansprüche

1. Eine Stoßwellenvorrichtung (100) umfassend eine Stoßwellenquelle (120),
**dadurch gekennzeichnet, dass**
die Stoßwellenquelle (120) mit einem Schwenkantrieb (140, 142) gekoppelt ist und für eine Schwenkbewegung (132) um mindestens eine Schwenkachse (130) konfiguriert ist, wobei der Schwenkantrieb (140, 142) mit einem Bewegungssensor (410) gekoppelt und für eine Schwenkbewegung (132) als Funktion eines Bewegungssensorsignals (412) des Bewegungssensors (410) ausgebildet ist, und der Bewegungssensor (410) mindestens einer von einem Atmungssensor oder einem Herzpulssensor ist.

2. Die Stoßwellenvorrichtung (100) nach Anspruch 1,
**dadurch gekennzeichnet, dass**
die Stoßwellenvorrichtung (100) ein Lithotripter umfassend einen Patiententisch (110) ist, wobei die Stoßwellenquelle (120) unterhalb des Patiententisches (110) angeordnet ist,
wobei der Patiententisch (110) eine Tischebene (114) und eine Längsachse (112) definiert,
die Stoßwellenquelle (120) für eine Schwenkbewegung (132) um eine zur Tischebene (114) parallele Schwenkachse (130) konfiguriert ist, und
der Bewegungssensor (410) ein Atmungssensor ist.

3. Die Stoßwellenvorrichtung (100) nach einem der vorhergehenden Ansprüche,
**dadurch gekennzeichnet, dass**
die Schwenkbewegung (132) auf einen Winkel kleiner als eines von 20°, 10°, 5°, 4°, 2° begrenzt ist.

4. Die Stoßwellenvorrichtung (100) nach Anspruch 2 oder nach Anspruch 3 in Kombination mit Anspruch 2,
**dadurch gekennzeichnet, dass**
die Stoßwellenquelle (120) von einer Schwenkhalterung (131) gehalten wird, die weiterhin so konfiguriert ist, dass sie um eine Achse (116) orthogonal zur Tischebene (114) rotiert, und/oder
die Schwenkhalterung (131) so konfiguriert ist, dass sie für eine feste Rotationsposition arretiert wird, wenn der Schwenkantrieb in Betrieb ist.

5. Die Stoßwellenvorrichtung (100) nach Anspruch 2 oder nach einem der Ansprüche 3-4 in Kombination mit Anspruch 2 mit Bezug auf den Atmungssensor (410),
**dadurch gekennzeichnet, dass**
der Atmungssensor (410) konfiguriert ist, um mit einem Patienten (800) gekoppelt zu werden, wobei der Patient auf dem Patiententisch positioniert ist.

6. Die Stoßwellenvorrichtung (100) nach Anspruch 2 oder nach einem der Ansprüche 3-5 in Kombination mit Anspruch 2,
**dadurch gekennzeichnet, dass**
die Stoßwellenquelle (120) konfiguriert ist, um Stoßwellen zu erzeugen, die sich in Richtung des Patiententisches (110) ausbreiten und über dem Patiententisch (110) ein Fokusvolumen (350) aufweisen.

7. Die Stoßwellenvorrichtung (100) nach Anspruch 2 oder nach einem der Ansprüche 3-6 in Kombination mit Anspruch 2,
**dadurch gekennzeichnet, dass**
die Stoßwellenquelle (120) konfiguriert ist, um das Fokusvolumen (350) durch Schwenken parallel zur Längsachse (112) des Patiententisches (110) mit einer rotationsbedingten Abweichung zu bewegen.

8. Die Stoßwellenvorrichtung (100) nach einem der vorhergehenden Ansprüche,
**dadurch gekennzeichnet, dass**
der Atmungssensor (410) konfiguriert ist, um mindestens eines der folgenden zu erfassen:
- einen mit einem Patienten (800) gekoppelten Atmungsluftstrom in und/oder aus dem Patienten (800),
- eine Brustbewegung des Patienten (800) aufgrund der Atmung, und
- eine Veränderung von Brustvolumen und/oder -größe des Patienten (800) aufgrund der Atmung,
- eine Veränderung der Lage eines inneren Organs wie Niere oder Herz unter Verwendung von Ultraschall-Bildgebung,
- eine Veränderung des Schwerpunkts eines Organs von Interesse.

9. Die Stoßwellenvorrichtung (100) nach einem der vorhergehenden Ansprüche,
**dadurch gekennzeichnet, dass**
mindestens ein Bildgebungssystem (450) vorgesehen ist und zum Erzeugen von Bildern eines Bereichs eines Patienten konfiguriert ist.

10. Die Stoßwellenvorrichtung (100) nach einem der vorhergehenden Ansprüche,
**dadurch gekennzeichnet, dass**
mindestens ein Controller/Computer (460) vorgesehen und konfiguriert ist, um mindestens eines der folgenden auszuführen:
- Berechnen eines optimalen Neigungswinkels und/oder Rotationswinkels,
- Visualisieren der Position eines Organs (810) und/oder eines Steins (820) zusammen mit einem Fokusvolumen (350) auf einem Bildschirm,
- Visualisieren der Bewegung eines Fokusvolumens (350) aufgrund einer Neigung,
- Visualisieren des gesamten durch die Neigung abgedeckten Fokusvolumens (310).

## Revendications

1. Dispositif à ondes de choc (100) comprenant une source d'ondes de choc (120),
**caractérisé en ce que**
la source d'ondes de choc (120) étant couplée à un entraînement d'inclinaison (140, 142) et configurée pour un mouvement d'inclinaison (132) autour d'au moins un axe d'inclinaison (130),
l'entraînement d'inclinaison (140, 142) étant couplé à un capteur de mouvement (410) et configuré pour un mouvement d'inclinaison (132) en fonction d'un signal de capteur de mouvement (412) provenant du capteur de mouvement (410), et le capteur de mouvement (410) est au moins un parmi un capteur de respiration ou un capteur de pouls cardiaque.

2. Dispositif à ondes de choc (100) selon la revendication 1,
**caractérisé en ce que**
le dispositif à ondes de choc (100) est un lithotriteur comprenant une table de patient (110), dans lequel la source d'ondes de choc (120) est agencée sous la table de patient (110),
la table de patient (110) définissant un plan de table (114) et un axe longitudinal (112),
la source d'ondes de choc (120) est configurée pour un mouvement d'inclinaison (132) autour d'un axe d'inclinaison (130) parallèle au plan de table (114), et
le capteur de mouvement (410) est un capteur de respiration.

3. Dispositif à ondes de choc (100) selon l'une quelconque des revendications précédentes, **caractérisé en ce que**
le mouvement d'inclinaison (132) est limité à un angle inférieur à un parmi : 20°, 10°, 5°, 4°, 2°.

4. Dispositif à ondes de choc (100) selon la revendication 2 ou selon la revendication 3 en combinaison avec la revendication 2,
**caractérisé en ce que**
la source d'ondes de choc (120) est maintenue par un support d'inclinaison (131) qui est en outre configuré pour tourner autour d'un axe (116) orthogonal au plan de table (114) et/ou
le support d'inclinaison (131) est configuré pour être verrouillé pour une position de rotation fixe lorsque l'entraînement d'inclinaison est opérationnel.

5. Dispositif à ondes de choc (100) selon la revendication 2 ou selon l'une quelconque des revendications 3 et 4 en combinaison avec la revendication 2 relative au capteur de respiration (410),
**caractérisé en ce que**
le capteur de respiration (410) est configuré pour être couplé à un patient (800), le patient étant positionné sur la table de patient.

6. Dispositif à ondes de choc (100) selon la revendication 2 ou selon l'une quelconque des revendications 3 à 5 en combinaison avec la revendication 2,
**caractérisé en ce que**
la source d'ondes de choc (120) est configurée pour générer des ondes de choc se propageant dans une direction vers la table de patient (110) et ayant un volume focal (350) au-dessus de la table de patient (110).

7. Dispositif à ondes de choc (100) selon la revendication 2 ou selon l'une quelconque des revendications 3 à 6 en combinaison avec la revendication 2,
**caractérisé en ce que**
la source d'ondes de choc (120) est configurée pour mettre en mouvement le volume focal (350) en l'inclinant parallèlement à l'axe longitudinal (112) de la table de patient (110) avec un écart causé par la rotation.

8. Dispositif à ondes de choc (100) selon l'une quelconque des revendications précédentes,
**caractérisé en ce que**
le capteur de respiration (410) est configuré pour capter au moins un parmi
- un flux d'air de respiration entrant et/ou sortant du patient (800) couplé à un patient (800),
- un mouvement thoracique du patient (800) dû à la respiration, et
- un changement du volume thoracique et/ou de la taille thoracique du patient (800) dû à la respiration
- un changement de position d'un organe interne tel que le rein ou le cœur en utilisant une imagerie par ultrasons,
- un changement du centre de gravité d'un organe d'intérêt.

9. Dispositif à ondes de choc (100) selon l'une quelconque des revendications précédentes,
**caractérisé en ce que**
au moins un système d'imagerie (450) est prévu et configuré pour générer des images d'une région d'un patient.

10. Dispositif à ondes de choc (100) selon l'une quelconque des revendications précédentes,
**caractérisé en ce que**
au moins un contrôleur/ordinateur (460) est prévu et configuré pour au moins un parmi :
- calculer un angle d'inclinaison optimal et/ou un angle de rotation optimal,
- visualiser sur un écran la position d'un organe (810) et/ou d'un calcul (820) conjointement avec un volume focal (350),
- visualiser le mouvement d'un volume focal (350) dû à l'inclinaison,
- visualiser le volume focal total (310) couvert par l'inclinaison.
